# NEUE EUROPÄISCHE PATENTSCHRIFT

(11) **EP 2 420 817 B2**
(45) Veröffentlichungstag und Bekanntmachung des Hinweises auf die Entscheidung über den Einspruch: **25.07.2018**
(45) Hinweis auf die Patenterteilung: 08.04.2015
(21) Anmeldenummer: 11163620.5
(22) Anmeldetag: 26.04.2011
(51) Int. Cl.: G01N 3/56, G01N 19/06

(54) **Prüfvorrichtung zur Überprüfung der Qualität einer Farb- oder Lackschicht**
Test device for testing the quality of a paint or varnish layer
Dispositif de contrôle pour la vérification de la qualité d'une couche de couleur ou de laque

(30) Priorität: 16.06.2010 DE 102010030159
(43) Veröffentlichungstag der Anmeldung: 22.02.2012
(73) Patentinhaber: Robert Bosch GmbH, 70442 Stuttgart (DE)
(72) Erfinder: Pniok, Raphael, 73732 Esslingen (DE); Eistert, Olaf, 73102 Birenbach (DE); Burk, Tobias, 72070 Tübingen (DE); Emmerich, Roland, 71717 Beilstein (DE)

(56) Entgegenhaltungen:
- CN-Y- 2 508 241
- DE-A1- 3 331 172
- DE-A1- 19 702 267
- GB-A- 2 443 530
- US-A- 5 985 987
- US-A1- 2004 051 543
- US-A1- 2009 078 035
- US-A1- 2009 320 575
- US-A1- 2009 320 575
- US-B2- 7 287 419
- Broschüre AFCONA vom Januar 2010
- Technical Bulletin "JEFFSPERSE X3503 Dispersant", April 2009

## Beschreibung

Die vorliegende Erfindung betrifft Prüfvorrichtung zur Überprüfung der Qualität einer Farb- oder Lackschicht und ein entsprechendes Verfahren.

### Stand der Technik

Bei Dispersionsfarben und Lacken entweichen nach der Applikation Lösemittel oder Wasser, sowie andere flüchtige Substanzen aus der noch feuchten Schicht. Da sich die Zusammensetzung des Lackes somit während der Trocknungsphase beständig ändert, kann eine Flokkulation (Reagglomerierung) der enthaltenen Pigmente auftreten. Ebenso kann eine Entmischung von Pigmenten mit unterschiedlicher Dichte, beispielsweise Titandioxid und Kupferphthalocyanin, auftreten. Im schlimmsten Fall bilden sich sechseckige Zellen, die sogenannten Bénardschen Zellen, die durch Strömungen innerhalb der Lackschicht verursacht werden.

Durch Reiben während des Trocknens werden Scherkräfte in das System eingebracht. So können die Agglomerate wieder zerteilt (deagglomeriert) beziehungsweise die Entmischung aufgehoben werden. Die geriebene Fläche kann also ein signifikant unterschiedliches, meist farbstärkeres Erscheinungsbild aufweisen als die nicht geriebene Fläche.

Der Unterschied zwischen einer geriebenen Fläche einer Farb- oder Lackschicht und einer nicht-geriebenen Referenzfläche der Farb- oder Lackschicht bezeichnet man als Rub-Out-Effekt. Dabei ist der Rub-Out-Effekt desto stärker, je farbstärker/farbschwächer die geriebene Fläche im Vergleich zur ungeriebenen Fläche erscheint. Erscheint die geriebene Fläche farbstärker als die ungeriebene Fläche, spricht man von einem positiven Rub-Out-Effekt, erscheint die geriebene Fläche farbschwächer von einem negativen Rub-Out-Effekt. Im Idealfall ist kein Unterschied in Farbstärke und -ton zu erkennen.

Der Rub-Out-Test wird herkömmlicherweise händisch durchgeführt, indem die prüfende Person einen blanken Finger, ein behandschuhter Finger oder einen Pinsel über den zu überprüfenden Prüfbereich reibt. Je nach Farb- oder Lacksystem kann das Reiben mit dem blanken Finger auf die Dauer jedoch ungesund sein. Demgegenüber steht die Tatsache, dass die Viskositätsgrenze mit Laborhandschuhen fast nicht spürbar ist. Bei einer Prüfung mit einem Pinsel, muss dieser aufwendig gereinigt werden, was unter anderem ein Grund dafür ist, dass diese Methode selten durchgeführt wird.

Zudem ist das Festlegen einer Prüfzeit problematisch. Zum Beispiel kann ein Rub-Out-Test mit dem blanken Finger bis zum "Ranziehen" des Lacks, das heißt bis zum Erreichen einer bestimmten, vom Prüfer wahrgenommenen Grenzviskosität des Lacks, durchgeführt. Dies gestaltet jedoch das prüferunabhängige Finden eines Endpunktes als äußerst schwierig. Das Festlegen einer definierten Prüfzeit, beispielsweise um den Gesamtenergieeintrag während der Prüfung zu standardisieren, berücksichtigt hingegen weder Unterschiede zwischen den Prüfern, noch systembedingt unterschiedliche Trocknungszeiten, da das Aufbrechen der Flokkulate nur dann effektiv ist, wenn es auch durch die fortgeschrittene Trocknung stabilisiert wird.

Letztendlich wurden die Bedingungen, unter welchen ein Rub-Out-Test durchzuführen ist, nicht normativ festgelegt. In den meisten Fällen ist das Ergebnis des Rub-Out-Tests stark vom Prüfer und dessen Tagesform abhängig. Zudem sind die Ergebnisse unterschiedlicher Labore aufgrund von unterschiedlichen Vorgehensweisen in den seltensten Fällen vergleichbar. Ansätze, wie die Überprüfung der Qualität einer Farb- oder Lackschicht automatisiert werden kann werden in den Druckschriften US 2,316,518 A1, US 2,287,148 A, US 3,040,559 A und US 3,974,678 beschrieben.

### Offenbarung der Erfindung

Gegenstand der vorliegenden Erfindung ist eine Prüfvorrichtung gemäß Anspruch 1 zur, insbesondere automatischen, Überprüfung der Qualität einer (Dispersions)-Farb- oder Lackschicht, welche mindestens eine Reibkörperbewegungseinrichtung umfasst. Erfindungsgemäß kann die Reibkörperbewegungseinrichtung dazu ausgebildet sein, mindestens einen Reibkörper, insbesondere automatisch, in Reibkontakt entlang einer definierten Bahn über einen Prüfbereich einer Farb- oder Lackschicht zu bewegen und/oder den Reibkontakt zwischen dem Reibkörper und dem Prüfbereich während der (andauernden) Bewegung des Reibkörpers, insbesondere automatisch, aufzuheben.

Eine Reibkörperbewegungseinrichtung hat die Vorteile, dass das Rub-Out-TestVerfahren standardisiert, personenabhängigen Abweichungen vermieden und die Reproduzierbarkeit der Prüfergebnisse erhöht werden kann. Zudem kann durch eine Reibkörperbewegungseinrichtung vorteilhafterweise der direkte Kontakt des Bedieners mit der Farb- oder Lackformulierung vermieden werden (Gesundheitsschutz).

Das Aufheben des Reibkontaktes zwischen dem Reibkörper und dem Prüfbereich während der Bewegung des Reibkörpers hat den Vorteil, dass Abdrücke auf dem Prüfbereich verhindert werden. Dies hat wiederum den Vorteil, dass die Vergleichbarkeit derartiger Prüfbereiche erhöht, Fehler durch einen durchscheinenden Untergrund (Schichtdickenschwankungen) vermieden werden und damit die Qualitätsüberprüfung verbessert wird.

Bei der Prüfvorrichtung kann es sich um eine halbautomatische oder vollautomatische Prüfvorrichtung für den Einsatz im Labor oder den Einsatz in einer Anlage zur Hochdurchsatzforschung (Hochdurchsatzanlage) handeln. Zum Beispiel kann die Prüfvorrichtung eine halbautomatische Prüfvorrichtung, zum Beispiel für den Einsatz im Labor, sein, welche beispielsweise manuell mit Reibkörpern und Farb-oder Lack-beschichteten Substraten bestückt wird. Die Prüfvorrichtung kann jedoch auch eine vollautomatische Prüfvorrichtung, zum Beispiel für den Einsatz in einer Anlage zur Hochdurchsatzforschung (Hochdurchsatzanlage), sein, welche beispielsweise die Farb- oder Lackherstellung, die Substratbestückung, die Substratbeschichtung, die Reibkörperbestückung, die Reibkörperbewegung, gegebenenfalls die Reibkörperreinigung, und die Auswertung automatisch durchführt. Insbesondere die Reibkörperbewegungseinrichtung der Prüfvorrichtung kann für Temperaturen ausgelegt sein, welche größer oder gleich dem Schmelz- beziehungsweise Verflüssigungstemperaturbereich von Pulverlacken, sind. Beispielsweise kann die Reibkörperbewegungseinrichtung für den Einsatz bis zu einer Temperatur von 220° ausgelegt sein. Auf diese Weise kann mit der Prüfvorrichtung auch die Qualität von Pulverlacken, welche nur bei erhöhten Temperaturen flüssig vorliegen, Überprüft werden.

Der Reibkontakt zwischen dem Reibkörper und dem Prüfbereich kann während der Bewegung des Reibkörpers dadurch aufgehoben werden, dass der Reibkörper während der (andauernden) Bewegung aus dem Prüfbereich heraus bewegt und/oder von dem Prüfbereich abgehoben wird. Durch beide Alternativen als auch durch eine Kombination dieser Alternativen können Abdrücke auf dem Prüfbereich verhindert werden, was mit den bereits beschriebenen Vorteilen einhergeht.

Im Rahmen einer Ausführungsform ist daher die Reibkörperbewegungseinrichtung dazu ausgebildet ist, den Reibkörper während der (andauernder) Bewegung, insbesondere automatisch, aus dem Prüfbereich heraus zu bewegen.

Im Rahmen einer weiteren Ausführungsform ist daher die Reibkörperbewegungseinrichtung dazu ausgebildet, den Reibkörper während der (andauernden) Bewegung, insbesondere automatisch, von dem Prüfbereich abzuheben.

Im Rahmen einer weiteren Ausführungsform ist die Reibkörperbewegungseinrichtung dazu ausgebildet, den Reibkörper in Reibkontakt entlang einer definierten Bahn über einen Prüfbereich einer unbewegten/feststehenden Farb- oder Lackschicht zu bewegen. Dadurch, dass die Farb- oder Lackschicht nicht bewegt wird kann die Prüfvorrichtung vereinfacht werden.

Erfindungsgemäß ist die Reibkörperbewegungseinrichtung dazu ausgebildet, den Reibkörper in Reibkontakt entlang einer definierten, zumindest teilweise gekrümmten Bahn über einen Prüfbereich zu bewegen. Auf diese Weise kann die Bewegung der meist kreisförmigen Bewegung eines manuell durchgeführten Rub-Out-Tests nachempfunden werden, was die Vergleichbarkeit erhöht. Bei rein linearem Hin- und Herbewegen des Reibkörpers kann hingegen der noch flüssige Beschichtungsstoff zur Seite geschoben werden, was zu einer starken Abnahme der Schichtstärke und einem durchscheinen des Untergrunds und damit zu einer Verfälschung der Ergebnisse führen kann. Außerdem können sich Punkte auf dem Reibkörper auf Kreisbahnen mit unterschiedlichen Mittelpunkten bewegen, wodurch eine optimale Durchmischung der entmischten Pigmente beziehungsweise Zerteilung von Agglomeraten gewährleistet und die Messfläche vergrößert werden kann.

Im Rahmen einer weiteren Ausführungsform ist die Reibkörperbewegungseinrichtung derart ausgebildet, dass die Form der Reibkörperbahn, insbesondere automatisch, einstellbar ist. Auf diese Weise können individuelle und reproduzierbare Reibbewegungen (Rub-Out-Bewegung) erzielt werden. Erfindungsgemäß kann die Reibkörperbewegungseinrichtung derart ausgebildet sein, dass die Reibkörperbahn einen ersten, gekrümmten Bahnabschnitt im Prüfbereich und einen zweiten gradlinigen Bahnabschnitt aus dem Prüfbereich heraus umfasst. Erfindungsgemäß ist der erste, gekrümmte Bahnabschnitt spiralförmig oder weist die Form eines einfach oder mehrfach durchlaufenen Ovals, insbesondere Kreises oder Ellipse, auf. Der zweite, gradlinige Bahnabschnitt ist erfindungsgemäß ein Bahnabschnitt, welcher tangential von dem ersten, gekrümmten Bahnabschnitt ausgeht. Auf diese Weise kann ebenfalls ein Abdruck auf der Bewertungsstelle vermieden werden.

Im Rahmen einer weiteren Ausführungsform umfasst die Reibkörperbewegungseinrichtung mindestens einen, insbesondere automatischen, Greifer zum lösbaren, insbesondere automatischen, Befestigen eines Reibkörpers. Vorzugsweise ist der Greifer dazu ausgebildet, einen Reibkörper automatisch zu greifen, zu fixieren und wieder frei zugeben. Insbesondere kann der Greifer ein Sauggreifer sein.

Der Reibkörper ist vorzugsweise, insbesondere hinsichtlich der Form, Größe, Flexibilität, Härte und/oder Oberflächenqualität, derart ausgebildet, dass eine möglichst gute Zerteilung von Agglomeraten beziehungsweise Aufhebung einer Entmischung erzielt wird. Vorzugsweise ist der Reibkörper derart ausgebildet, dass die Farb- oder Lackschicht nicht seitlich verschoben wird. Auf diese Weise kann nach dem Reibkontakt eine deckende Farb- oder Lackschicht erhalten werden. Zum Beispiel kann der Reibkörper, insbesondere hinsichtlich der Flexibilität, Härte und/oder Oberflächenqualität, einem menschlichen Finger nachgebildet sein. Der Reibkörper kann jedoch auch ein pinselförmiger Körper oder ein Körper mit einer ebenen (flachen) Reibfläche, beispielsweise eine flache Scheibe, oder ein Körper mit einer konvexen Reibfläche, beispielsweise eine abgerundete/ausgewölbte Scheibe, ein Ovaloid, ein Halbovaloid, ein Ellipsoid, ein Halbellipsoid, eine Kugel oder eine Halbkugel, sein.

Im Rahmen einer weiteren Ausführungsform ist der Reibkörper ein Körper mit einer konvexen Reibfläche, beispielsweise eine abgerundete/ausgewölbte Scheibe, ein Ovaloid oder ein Halbovaloid, insbesondere ein Ellipsoid, ein Halbellipsoid, eine Kugel oder eine Halbkugel.

Im Rahmen einer weiteren Ausführungsform ist der Reibkörper reinigbar, insbesondere automatisch reinigbar, oder ein Einweg-Teil. Die Verwendung von Einweg-Reibkörpern hat die Vorteile, dass keine Reinigung erforderlich ist sowie schnelle und prozesssichere Farbwechsel vorgenommen werden können.

Im Rahmen einer weiteren Ausführungsform ist die Reibkörperbewegungseinrichtung derart ausgebildet, dass die Auflagekraft, mit welcher der Reibkörper auf der Farb- oder Lackschicht aufliegt, insbesondere automatisch und/oder reproduzierbar, einstellbar ist. Auf diese Weise kann ein Rub-Out-Test mit unterschiedlichen Auflagekräften an einer Farb- oder Lackschicht durchgeführt werden. Zudem kann so der Rub-Out-Test an unterschiedliche Farb- oder Lackformulierungen und/oder -schichtdicken angepasst werden.

Beispielsweise kann die Auflagekraft durch ein oder mehrere, montierbare oder einstellbare Gewichte und/oder eine oder mehrere Federn und/oder einen Motor und/oder einen Zylinder einstellbar sein. Insbesondere beim Einsatz von Federn, kann weiterhin mindestens ein Schwingungsdämpfer eingesetzt werden, um Feder bedingte Schwingungen zu dämpfen.

Im Rahmen einer weiteren Ausführungsform ist die Reibkörperbewegungseinrichtung derart ausgebildet, dass die Reibzeit (Rub-Out-Zeit), während welcher der mindestens eine Reibkörper in Reibkontakt mit dem Prüfbereich steht, insbesondere automatisch und/oder reproduzierbar, einstellbar ist. Auf diese Weise kann der Gesamtenergieeintrag standardisiert und reproduziert werden. Vorzugsweise ist die Reibkörperbewegungseinrichtung dazu ausgebildet, den Reibkörper während einer Bewegung des Reibkörpers auf den Prüfbereich der Farb- oder Lackschicht, insbesondere automatisch, aufzusetzen. Auf diese Weise können ebenfalls Abdrücke auf dem Prüfbereich verhindert werden.

Im Rahmen einer weiteren Ausführungsform umfasst die Reibkörperbewegungseinrichtung mindestens einen Kraftsensor zum Messen einer beim Reibkontakt auf den Reibkörper seitlich wirkenden Kraft. Beispielsweise kann die Reibkörperbewegungseinrichtung für jeden Reibkörper mindestens einen Kraftsensor zum Messen einer auf den Reibkörper seitlich wirkenden Kraft umfassen. Durch derartige Kraftsensoren kann zum Beispiel der Trocknungszustand der Farb- oder Lackschicht erkannt und die Reibzeit an die Farb- oder Lackschichtformulierung, den Reibkörper und/oder das Substrat angepasst werden.

Insbesondere kann die Prüfvorrichtung mehrere Reibkörper zum Überprüfen der Qualität einer Farb- oder Lackschicht in mehreren Prüfbereichen umfassen. Auf diese Weise können parallel mehrere Rub-Out-Tests unter identischen Bedingungen (Zeiten, Bewegungsbahnen) auf einem oder mehreren Substraten mit gleichen oder unterschiedlichen Reibkörpern, zum Beispiel zur Vergleichsprüfung einer Probe und einer Referenzformulierung, durchgeführt werden.

Im Rahmen einer Ausführungsform ist daher die Reibkörperbewegungseinrichtung dazu ausgebildet, mindestens einen ersten und mindestens einen zweiten Reibkörper jeweils in Reibkontakt entlang einer definierten Bahn über einen Prüfbereich zu bewegen. Zum Beispiel können der oder die ersten Reibkörper jeweils in Reibkontakt entlang einer definierten Bahn über einen Prüfbereich einer Farb- oder Lackschicht und der oder die zweiten Reibkörper jeweils in Reibkontakt entlang einer definierten Bahn über einen Prüfbereich einer Referenz bewegt werden. Insbesondere kann die Reibkörperbewegungseinrichtung dabei dazu ausgebildet sein, mehrere erste Reibkörper jeweils in Reibkontakt entlang einer definierten Bahn über einen Prüfbereich einer Farb- oder Lackschicht und/oder einen oder mehrere zweite Reibkörper jeweils in Reibkontakt entlang einer definierten Bahn über einen Prüfbereich einer Referenz, insbesondere einer Referenzfarbschicht oder Referenzlackschicht, zu bewegen.

Im Rahmen einer Ausgestaltung ist dabei die Form der Reibbahnen und die Reibzeit der ersten und/oder zweiten Reibkörper gleich oder zumindest im Wesentlichen gleich. Insbesondere kann die Reibkörperbewegungseinrichtung dazu ausgebildet sein, die ersten und zweiten Reibkörper gleichzeitig zu bewegen. Auf diese Weise kann die Qualität einer Vielzahl von Farb- oder Lackschichten unter gleichen und reproduzierbaren Bedingungen überprüft und mit der Referenz verglichen werden. Die Auflagekraft der Reibkörper kann dabei grundsätzlich ebenfalls gleich oder zumindest im Wesentlichen gleich sein.

Im Rahmen einer besonderen Ausgestaltung ist jedoch die Auflagekraft der einzelnen Reibkörper unterschiedlich einstellbar. Insbesondere kann die Auflagekraft der ersten Reibkörper untereinander und/oder der zweiten Reibkörper untereinander unterschiedlich einstellbar sein. Dies kann beispielsweise dadurch gewährleistet werden, dass die Reibkörper jeweils einzeln gelagert sind und/oder dass die Reibkörperbewegungseinrichtung für jeden Reibkörper ein individuelles, gewichtsbasiertes, federbasiertes und/oder motorisches Auflagekrafteinstellsystem umfasst. Dies hat zum Beispiel einerseits den Vorteil, dass der Einfluss der Auflagekraft auf die Qualität einer Farb- oder Lackschicht beziehungsweise Farb-oder Lackformulierung untersucht werden kann. Andererseits hat dies zum Beispiel den Vorteil, dass die Überprüfungsbedingungen individuell an unterschiedliche, beispielsweise unterschiedlich dicke, Farb- oder Lackschichten beziehungsweise unterschiedliche Farb- oder Lackformulierungen angepasst werden können und auf diese Weise die Qualität mehrerer unterschiedlicher Farb- oder Lackschichten beziehungsweise -formulierungen gleichzeitig in einem Arbeitsschritt überprüft werden kann.

Alternativ oder zusätzlich dazu, kann die Prüfvorrichtung zwei oder mehr Reibkörperbewegungseinrichtungen umfassen.

Im Rahmen einer weiteren Ausführungsform umfasst die Prüfvorrichtung zwei oder mehr Reibkörperbewegungseinrichtungen, wobei die Reibkörperbewegungseinrichtungen jeweils dazu ausgebildet sind, mindestens einen Reibkörper in Reibkontakt entlang einer definierten Bahn über einen Prüfbereich einer Farb-oder Lackschicht zu bewegen und den Reibkontakt zwischen dem Reibkörper und dem Prüfbereich während der (andauernden) Bewegung des Reibkörpers aufzuheben. Dabei können die Reibkörperbewegungseinrichtungen jeweils dazu ausgebildet sein, nur einen Reibkörper, nur einen ersten und einen zweiten Reibkörper, oder einen oder mehrerer erste und einen oder mehrere zweite Reibkörper jeweils in Reibkontakt entlang einer definierten Bahn über einen Prüfbereich zu bewegen. Zum Beispiel können die Reibkörperbewegungseinrichtungen jeweils dazu ausgebildet sein, den oder die ersten Reibkörper jeweils in Reibkontakt entlang einer definierten Bahn über einen Prüfbereich einer Farb-oder Lackschicht und den oder die zweiten Reibkörper jeweils in Reibkontakt entlang einer definierten Bahn über einen Prüfbereich einer Referenz zu bewegen. Insbesondere können die Reibkörperbewegungseinrichtungen dabei jeweils dazu ausgebildet sein, mehrere erste Reibkörper jeweils in Reibkontakt entlang einer definierten Bahn über einen Prüfbereich einer Farb- oder Lackschicht und/oder einen oder mehrere zweite Reibkörper jeweils in Reibkontakt entlang einer definierten Bahn über einen Prüfbereich einer Referenz, insbesondere einer Referenzfarbschicht oder Referenzlackschicht, zu bewegen.

Die Form der Reibbahnen und die Reibzeit der Reibkörper der einzelnen Reibkörperbewegungseinrichtungen kann dabei gleich oder verschieden sein. Darüber hinaus können die einzelnen Reibkörperbewegungsweinrichtungen dazu ausgebildet sein, ihre Reibkörper gleichzeitig oder zu unterschiedlichen Zeitpunkten zu bewegen. Auf diese Weise kann die Qualität einer Vielzahl von Farb- oder Lackschichten unter unterschiedlichen, reproduzierbaren Bedingungen, beispielsweise nach unterschiedlichen Trocknungszeiten, überprüft werden.

Die Auflagekraft der Reibkörper der einzelnen Reibkörperbewegungseinrichtungen kann dabei ebenfalls grundsätzlich gleich sein. Im Rahmen einer besonderen Ausgestaltung ist jedoch die Auflagekraft der Reibkörper der einzelnen Reibkörperbewegungseinrichtungen unterschiedlich einstellbar. Dies kann beispielsweise dadurch gewährleistet werden, dass die Reibkörperbewegungseinrichtungen jeweils einzeln gelagert sind und/oder ein individuelles, gewichtsbasiertes, federbasiertes und/oder motorisches Auflagekrafteinstellsystem aufweisen. Zusätzlich kann die Auflagekraft der einzelnen Reibkörper innerhalb einer Reibkörperbewegungseinrichtung unterschiedlich einstellbar sein. Dies kann - wie bereits erläutert - dadurch gewährleistet werden, dass die Reibkörper jeweils einzeln gelagert sind und/oder ein individuelles, gewichtsbasiertes, federbasiertes und/oder motorisches Auflagekrafteinstellsystem aufweisen.

Weiterhin kann die Prüfvorrichtung eine Substratfixierungseinrichtung umfassen. Beispielsweise kann die Substratfixierungseinrichtung ein Grundkörper mit einer oder mehreren Aussparungen zur Aufnahme von Substraten, insbesondere Prüfkarten, umfassen. Durch Einlegen jeweils eines Substrats in eine Aussparung kann zum Beispiel gewährleistet werden, dass eine auf dem Substrat aufgebrachte Farb- oder Lackschicht während der Reibbewegung des Reibkörpers unbewegt bleibt. Alternativ oder zusätzlich dazu kann die Substratfixierungseinrichtung eine oder mehrere Klemmen zum Festklemmen von Substraten umfassen.

Die Prüfvorrichtung kann weiterhin eine Beschichtungseinrichtung zum automatischen Beschichten von Substraten, beispielsweise Prüfkarten, mit einer oder mehreren, gleichen oder unterschiedlichen Farb- oder Lackschicht umfassen. Die Beschichtungseinrichtung kann beispielsweise eine Farb- oder Lackschicht durch ein Rakel-, Spritz-, Walz- und/oder Schleuder-Verfahren automatisch auf das Substrat aufbringen.

Außerdem kann die Prüfvorrichtung eine Farb- oder Lackherstellungseinrichtung zum automatischen Herstellen/Mischen von Farb- oder Lackformulierungen umfassen.

Zudem kann die Prüfvorrichtung eine Substratbestückungseinrichtung zum Bestücken der Prüfvorrichtung mit einem oder mehreren Substraten und zum Entfernen der beschichteten und geprüften Substrate umfassen. Dabei kann die Substratbestückungseinrichtung dazu ausgebildet sein, eines oder mehrere Substrate aus einem Substratbereitstellungsreservoir automatisch zu entnehmen und nach der Durchführung der Prüfung automatisch in ein Substratsammelreservoir zu entlassen.

Darüber hinaus kann die Prüfvorrichtung ein Reibkörperbereitstellungsreservoir zur Bereitstellung von Reibkörpern zur Durchführung der Prüfung und/oder ein Reibkörpersammelreservoir zum Sammeln von Reibkörpern nach der Durchführung der Prüfung umfassen. Der oder die Greifer können dabei dazu ausgebildet sein, jeweils einen Reibkörper aus dem Reibkörperbereitstellungsreservoir automatisch zu greifen und nach der Durchführung der Prüfung in das Reibkörpersammelreservoir zu entlassen. Das Reibkörpersammelreservoir kann derart ausgebildet sein, dass die Reibkörper darin automatisch gereinigt werden und anschließend wieder den Reibkörperbereitstellungsreservoir zugeführt werden. Die Prüfvorrichtung kann jedoch auch eine Reibkörperreinigungseinrichtung umfassen, welche ausgestaltet ist, Reibkörper dem Reibkörpersammelreservoir automatisch zu entnehmen, automatisch zu reinigen und automatisch dem Reibkörperbereitstellungsreservoir zuzuführen. Es ist jedoch ebenso möglich das Reibkörperbereitstellungsreservoir mit Einweg-Reibkörpern zu bestücken und die benutzten, im Reibkörpersammelreservoir gesammelten Einweg-Reibkörper zu entsorgen. So kann der personelle Reinigungsaufwand minimiert werden.

Zudem kann die Prüfvorrichtung eine Auswertungseinrichtung zum Auswerten des Einflusses des Reibkontakts auf den/die Prüfbereich/e, beispielsweise mittels Farb- und/oder Glanzmessung, umfassen.

Ein weiterer Gegenstand der vorliegenden Erfindung ist ein Prüfverfahren nach Anspruch 12 zur Überprüfung, insbesondere zur automatischen Überprüfung, der Qualität einer (Dispersions)-Farb- oder Lackschicht, insbesondere mit einer erfindungsgemäßen Prüfvorrichtung. Erfindungsgemäß kann in diesem Verfahren mindestens ein Reibkörper in Reibkontakt entlang einer definierten Bahn über einen Prüfbereich einer Farb- oder Lackschicht automatisch bewegt werden und/oder der Reibkontakt zwischen dem Reibkörper und dem Prüfbereich während der (andauernden) Bewegung des Reibkörpers automatisch aufgehoben wird.

Hinsichtlich der Vorteile und zusätzlicher Merkmale des Prüfverfahrens wird hiermit explizit auf die im Zusammenhang mit der Prüfvorrichtung erläuterten Vorteile und Merkmale verwiesen.

Insbesondere kann der Reibkörper automatisch in Reibkontakt entlang einer definierten, zumindest teilweise gekrümmten Bahn über den Prüfbereich bewegt werden.

Alternativ oder zusätzlich dazu kann der Reibkörper im Rahmen des Prüfverfahrens automatisch in Reibkontakt entlang einer definierten Bahn über einen Prüfbereich einer unbewegten/feststehenden Farb- oder Lackschicht bewegt werden. Die Form der Reibkörperbahn kann im Rahmen des Prüfverfahrens automatisch eingestellt werden. Erfindungsgemäß umfasst die Reibkörperbahn einen ersten, gekrümmten Bahnabschnitt im Prüfbereich und einen zweiten gradlinigen Bahnabschnitt aus dem Prüfbereich heraus. Erfindungsgemäß ist der erste, gekrümmte Bahnabschnitt spiralförmig oder weist die Form eines einfach oder mehrfach durchlaufenen Ovals, insbesondere Kreises oder Ellipse, auf. Der zweite, gradlinige Bahnabschnitt ist erfindungsgemäß ein Bahnabschnitt, welcher tangential von dem ersten, gekrümmten Bahnabschnitt ausgeht.

Im Rahmen einer weiteren Ausführungsform wird der Reibkontakt zwischen dem Reibkörper und dem Prüfbereich aufgehoben, in dem der Reibkörper während der (andauernden) Bewegung, insbesondere automatisch, aus dem Prüfbereich heraus bewegt, und/oder von dem Prüfbereich abgehoben wird. Zum Beispiel kann der Reibkörper nach dem Ablauf einer definierten Reibzeit (t_{R}) oder Überschreitung einer definierten, seitlichen Kraft (F_{R}, Antrocknen des Lacks) auf den Reibkörper, beispielsweise tangential, aus der Prüfposition/Reibposition gefahren und außerhalb der Reibposition abgehoben werden.

Grundsätzlich kann die Bewegung des Reibkörpers beim oder nach dem in Kontakt bringen mit dem Prüfbereich gestartet werden. Die Bewegung des Reibkörpers kann jedoch auch schon vor einem in Kontaktbringen mit dem Prüfbereich beziehungsweise Aufsetzen auf den Prüfbereich gestartet werden, wobei der bewegte Reibkörper mit dem Prüfbereich in Kontakt gebracht wird. Dies hat den Vorteil, dass Abdrücke im Prüfbereich vermieden werden können.

Der bewegte Reibkörper kann dabei automatisch auf den Prüfbereich aufgesetzt werden. Insbesondere kann der Reibkörper mit einer definierten Auflagekraft auf den Prüfbereich aufgesetzt werden. Die definierte Auflagekraft (F_{A}) kann dabei automatisch eingestellt werden.

Im Rahmen einer weiteren Ausführungsform wird die Farb- oder Lackschicht automatisch auf ein Substrat, beispielsweise eine Prüfkarte, aufgebracht. Dies kann beispielsweise durch ein Rakel-, Spritz-, Walz- und/oder Schleuder-Verfahren automatisch auf das Substrat erfolgen. Die Farb- oder Lackformulierung für die Farb- oder Lackschicht kann dafür ebenfalls automatisch hergestellt beziehungsweise gemischt werden.

Im Rahmen einer weiteren Ausführungsform wird der Reibkörper nach einer definierten Wartezeit/Trocknungszeit (t_{T}), ab dem Aufbringen der Farb- oder Lackschicht, automatisch in Kontakt mit dem Prüfbereich gebracht. Die Wartezeit/Trocknungszeit (t_{T}) kann dabei ebenfalls automatisch eingestellt werden.

Im Rahmen einer weiteren Ausführungsform wird der Reibkörper eine definierte Reibzeit (t_{R}) lang in Reibkontakt über den Prüfbereich bewegt. Die Reibzeit (t_{R}) kann dabei ebenfalls automatisch eingestellt werden. Nach Ablauf der definierten Reibzeit (t_{R}) kann der Prüfbereich automatisch ausgewertet werden.

Nach dem Aufheben des Reibkontakts kann der Reibkörper, insbesondere automatisch, gereinigt und/oder, beispielsweise gegen einen gereinigten Reibkörper oder Einweg-Reibkörper, ausgetauscht werden.

Im Rahmen einer weiteren Ausführungsform wird die beim Reibkontakt seitlich auf den Reibkörper wirkende Kraft (F_{R}) gemessen und als Maß für den Zustand, insbesondere den Trocknungszustand, der Farb- oder Lackschicht und/oder als Maß für das Aufheben/Beenden des Reibkontaktes, insbesondere an einem definierten Punkt, verwendet. Auf diese Weise kann die Qualitätsprüfung (Rub-Out-Test) beziehungsweise die Reibzeit (Rub-Out-Zeit) automatisch an unterschiedliche Farb- oder Lackformulierungen, Reibkörper und Substrate angepasst werden.

Der Reibkörper kann, beispielsweise durch einen automatischen Greifer, insbesondere einen automatischen Sauggreifer, zu Beginn des Prüfverfahrens automatisch gegriffen, während des Prüfverfahrens automatisch fixiert/gehalten und zum Abschluss des Prüfverfahrens automatisch frei gegeben werden.

Insbesondere können im Rahmen des Prüfverfahrens die Qualität von mehreren Farb- oder Lackschichten durch eine Reibbewegung von mehren Reibkörpern in mehreren Prüfbereichen überprüft und mit Referenzen verglichen werden. Dafür kann zum Beispiel mindestens ein erster und mindestens ein zweiter Reibkörper jeweils in Reibkontakt entlang einer definierten Bahn über einen Prüfbereich bewegt werden. Zum Beispiel können der oder die ersten Reibkörper jeweils in Reibkontakt entlang einer definierten Bahn über einen Prüfbereich einer Farb-oder Lackschicht und der oder die zweiten Reibkörper jeweils in Reibkontakt entlang einer definierten Bahn über einen Prüfbereich einer Referenz, insbesondere einer Referenzfarbschicht oder Referenzlackschicht, bewegt werden. Die Form der Reibbahnen und die Reibzeit der Reibkörper kann dabei gleich oder verschieden sein. Darüber hinaus können die Reibkörper gleichzeitig oder zu unterschiedlichen Zeitpunkten bewegt werden. Die Auflagekraft der Reibkörper kann dabei grundsätzlich gleich sein. Im Rahmen einer besonderen Ausgestaltung ist jedoch die Auflagekraft der einzelnen Reibkörper unterschiedlich einstellbar.

Das Prüfverfahren kann halbautomatisch oder vollautomatisch, im Labor oder in einer Anlage zur Hochdurchsatzforschung (Hochdurchsatzanlage) durchgeführt werden. Zum Beispiel kann das Prüfverfahren halbautomatisch, zum Beispiel im Labor, durchgeführt werden, wobei beispielsweise das Bestücken mit Reibkörpern und farb- oder lack-beschichteten Substraten manuell erfolgt. Das Prüfverfahren kann jedoch auch vollautomatisch, zum Beispiel in einer Anlage zur Hochdurchsatzforschung (Hochdurchsatzanlage), durchgeführt werden, wobei die Farb- oder Lackherstellung, die Substratbestückung, die Substratbeschichtung, die Reibkörperbestückung, die Reibkörperbewegung, gegebenenfalls die Reibkörperreinigung, und die Auswertung automatisch durchführt werden. Weiterhin kann im Rahmen des Prüfverfahrens der Einfluss des Reibkontakts auf den/die Prüfbereich/e, beispielsweise durch eine Auswertungseinrichtung, zum Beispiel mittels Farb- und/oder Glanzmessung, insbesondere automatisch ausgewertet werden.

### Zeichnungen und Beispiele

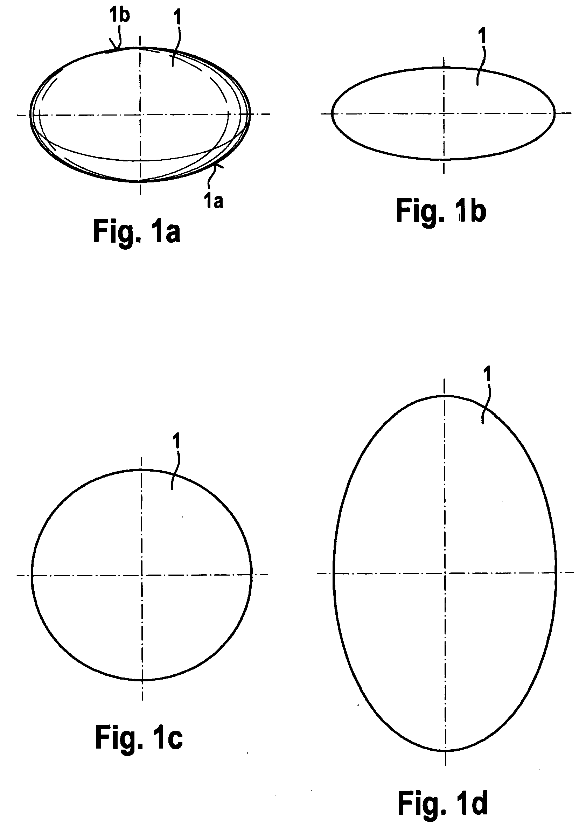

Weitere Vorteile und vorteilhafte Ausgestaltungen der erfindungsgemäßen Gegenstände werden durch die Zeichnungen veranschaulicht und in der nachfolgenden Beschreibung erläutert. Dabei ist zu beachten, dass die Zeichnungen nur beschreibenden Charakter haben und nicht dazu gedacht sind, die Erfindung in irgendeiner Form einzuschränken. Es zeigen
- Fig. 1a: eine schematische, perspektivische Ansicht einer Ausführungsform eines ovaloiden Reibkörpers;
- Fig. 1b: einen schematischen Querschnitt durch die in Figur 1a gezeigte Ausführungsform eines Reibkörpers senkrecht zur Reibebene;
- Fig. 1 c: eine Alternative der in Figur 1a und 1b gezeigten Ausführungsform eines Reibkörpers mit einem parallel zur Reibebene kreisförmig ausgebildeten Querschnitt;
- Fig. 1 d: eine andere Alternative der in Figur 1a und 1b gezeigten Ausführungsform eines Reibkörpers mit einem parallel zur Reibebene oval ausgebildeten Querschnitt;
- Fig. 2: einen schematischen Querschnitt durch eine Ausführungsform eines Reibkörpers in Form eines Halbovaloids beziehungsweise einer einseitig ausgewölbten Scheibe;
- Fig. 3: eine schematische Draufsicht auf zwei Farb- oder Lackschichten zur Veranschaulichung von Ausführungsformen der Reibkörperbewegung;
- Fig. 4a: einen schematischen Querschnitt durch eine erste Ausführungsform einer Reibkörperbewegungseinrichtung;
- Fig. 4b: einen schematischen Querschnitt durch eine zweite Ausführungsform einer Reibkörperbewegungseinrichtung;
- Fig. 4c: einen schematischen Querschnitt durch eine dritte Ausführungsform einer Reibkörperbewegungseinrichtung;
- Fig. 4d: einen schematischen Querschnitt durch eine vierte Ausführungsform einer Reibkörperbewegungseinrichtung;
- Fig. 5: ein Flussdiagramm zur Veranschaulichung einer Ausführungsform des erfindungsgemäßen Verfahrens.

Figur 1a zeigt eine schematische, perspektivische Ansicht einer Ausführungsform eines ovaloiden Reibkörpers 1. Ein derartiger Reibkörper 1 kann auch als Reibkörper in Form einer beidseitig ausgewölbten Scheibe bezeichnet werden. Figur 1a zeigt, dass der Reibkörper 1 eine konvexe Reibfläche 1a aufweist. Figur 1 zeigt zudem, dass auch die Fläche 1 b des Reibkörpers 1, welche der Reibfläche 1a gegenüberliegt, konvex ausgestaltet ist. Dies wird ebenfalls durch den in Figur 1b gezeigten, schematischen Querschnitt (senkrecht zur Reibebenen) durch diese Ausführungsform verdeutlicht. Ein derartiger Reibkörper 1 kann beispielsweise von einem Sauggreifer gegriffen, fixiert und wieder frei gegeben werden.

Die Figuren 1c und 1d veranschaulichen, dass der ovaloide Reibkörper 1 aus den Figuren 1a und 1b parallel zur Reibebene unterschiedlich ausgestaltete Querschnitte aufweisen kann. Figur 1c zeigt dabei eine parallel zur Reibebene kreisförmig ausgestaltete Alternative und Figur 1d eine parallel zur Reibebene oval ausgestaltete Alternative.

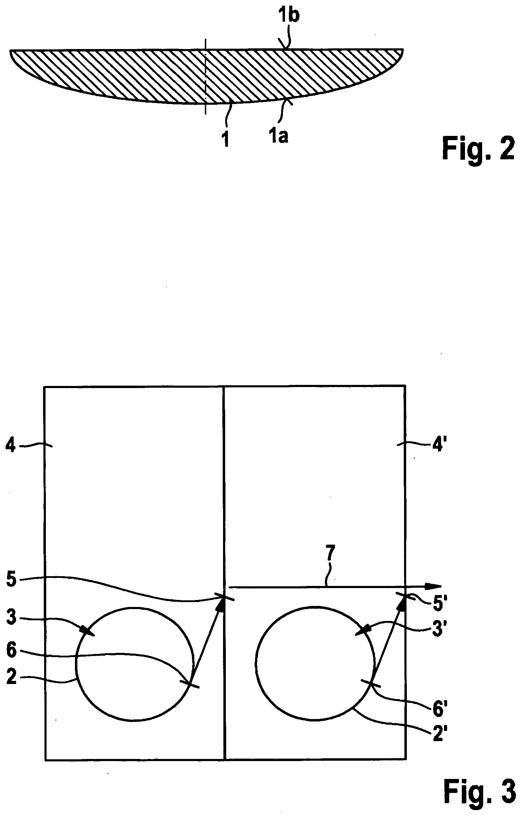

Figur 2 zeigt einen schematischen Querschnitt durch eine Ausführungsform eines Reibkörpers 1 in Form eines Halbovaloids beziehungsweise einer einseitig ausgewölbten Scheibe. Figur 2 zeigt, dass auch dieser Reibkörper 1 eine konvexe Reibfläche 1a aufweist. Im Gegensatz zu dem in den Figuren 1a bis 1d gezeigten Reibkörpern 1, ist hierbei jedoch die Fläche 1b des Reibkörpers 1, welche der Reibfläche 1a gegenüberliegt, plan ausgestaltet. Ein derartiger Reibkörper 1 kann ebenfalls von einem Sauggreifer gegriffen, fixiert und wieder frei gegeben werden. Gegebenenfalls kann ein derartiger Reibkörper 1 auf der Fläche 1b, welche der Reibfläche 1a gegenüberliegt, einen Griffabschnitt aufweisen (nicht dargestellt), welcher von einem mechanischen Greifer ergriffen werden kann.

Figur 3 ist eine schematische Draufsicht auf zwei Farb- oder Lackschichten 4,4' und dient der Veranschaulichung möglicher Reibkörperbewegungen. Figur 3 zeigt, dass pro Farb- oder Lackschicht 4,4' jeweils ein Reibkörper (nicht dargestellt) zunächst in Reibkontakt entlang eines kreisförmigen Bahnabschnittes 2 in einen Prüfbereich 3,3' einer Farb- oder Lackschichten bewegt wird und anschließend der Reibkontakt zwischen dem Reibkörper und dem Prüfbereich 3 während der Bewegung des Reibkörpers aufgehoben wird.

Dies kann zum Beispiel - wie mit dem Bezugszeichen 5,5' gekennzeichnet - dadurch erfolgen, dass der Reibkörper nach dem einfachen oder mehrfachen durchlaufen des kreisförmigen Bahnabschnitts 2,2' während der andauernden Bewegung tangential 5,5' aus dem Prüfbereich 3,3' heraus bewegt und außerhalb des Prüfbereichs 3,3' von der Farb- oder Lackschicht 4,4' abgehoben wird.

Wie mit den Bezugseichen 6,6' gekennzeichnet, ist es jedoch ebenso möglich, dass der Reibkörper nach dem einfachen oder mehrfachen durchlaufen des kreisförmigen Bahnabschnitts 2,2' während der andauernden Bewegung von dem Prüfbereich 3,3' abgehoben wird.

Nach dem Abheben von der Farb- oder Lackschicht 4,4' können die Prüfkörper durch eine Translation in einer Richtung 7 von den Farb- oder Lackschicht 4,4' weg bewegt werden, beispielsweise um gereinigt oder entsorgt zu werden.

Um eine in Figur 3 veranschaulichte Reibbewegung zu Erzielen kann die Prüfvorrichtung entweder eine Reibkörperbewegungseinrichtung, welche dazu ausgebildet ist, mindestens einen ersten und mindestens einen zweiten Reibkörper jeweils in Reibkontakt entlang einer definierten Bahn 2,2 über einen Prüfbereich 3,3' zu bewegen, oder mindestens zwei Reibkörperbewegungseinrichtungen, welche jeweils dazu ausgebildet sind, mindestens einen Reibkörper in Reibkontakt entlang einer definierten Bahn 2,2' über einen Prüfbereich 3,3' zu bewegen, umfassen.

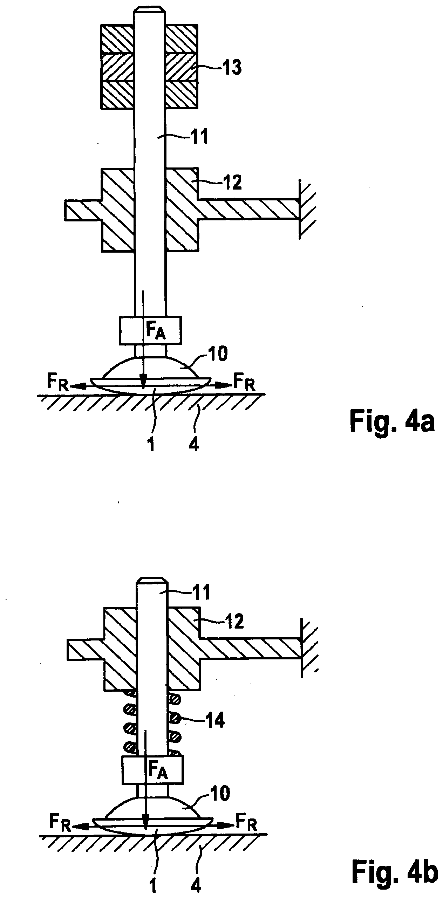

Die Figuren 4a bis 4d zeigen schematische Querschnitte durch eine erste bis vierte Ausführungsform einer Reibkörperbewegungseinrichtung. Die Figuren zeigen, dass die Reibkörperbewegungseinrichtungen einen Greifer 10, beispielsweise einen Sauggreifer, zum Greifen, Fixieren und frei geben eines Reibkörpers 1 umfassen. Dieser Greifer 10 ist an einem vertikal beweglichen Schaft 11 montiert, welcher durch eine Führung 12 vertikal zur untersuchenden Farb- oder Lackschicht 4 gehalten wird.

Im Rahmen der in Figur 4a gezeigten Ausführungsform sind an dem Schaft 11 Gewichte 13 befestigbar. Durch das Befestigen einer definierten Anzahl von Gewichten 13 kann eine definierte Auflagekraft F_{A} des Reibkörpers 1 auf der Farb-oder Lackschicht 4 eingestellt werden. Alternativ zum Befestigen einer definierten Anzahl von Gewichten 13 kann die Reibkörperbewegungseinrichtung eine gewichtsbasierte Auflagekraftstellungsvorrichtung (nicht dargestellt) umfassen. Die gewichtsbasierte Auflagekraftstellungsvorrichtung kann beispielsweise ein Gewicht umfassen, welches derart beweglich angeordnetes ist, dass durch eine Positionsveränderung des beweglichen Gewichts die Auflagekraft F_{A} des Reibkörpers 1 auf der Farb- oder Lackschicht 4 einstellbar ist. Figur 4a veranschaulicht ferner eine beim Reibkontakt auf den Reibkörper 1 seitlich wirkenden Kraft F_{R}, welche mit einem dafür ausgelegten Kraftsensor gemessen werden kann.

Die in Figur 4b gezeigte Ausführungsform unterscheidet sich dadurch von der in Figur 4a gezeigten Ausführungsform, dass anstelle von Gewichten 13 eine Feder 14 zwischen dem Greifer 10 und der Führung 12 angeordnet ist, um eine definierte Auflagekraft F_{A} des Reibkörpers 1 auf der Farb- oder Lackschicht 4 zu gewährleisten.

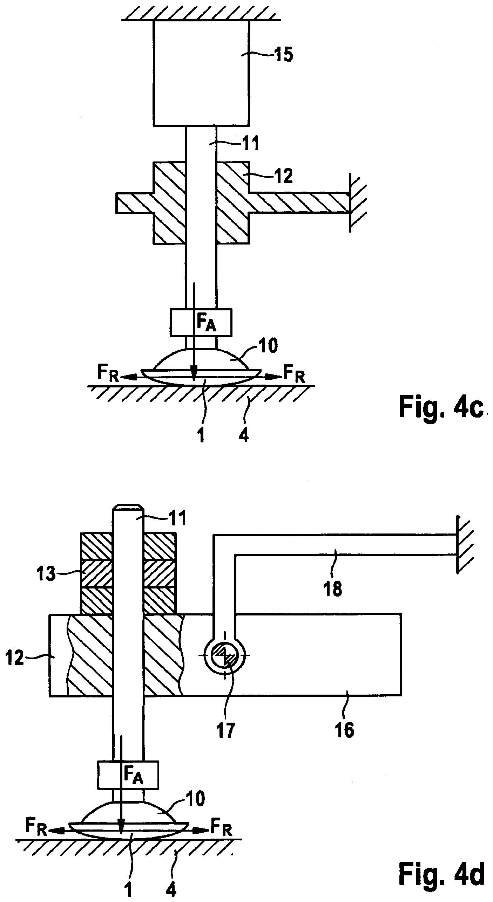

Die in Figur 4c gezeigte Ausführungsform unterscheidet sich dadurch von der in Figur 4a gezeigten Ausführungsform, dass anstelle von Gewichten 13 ein Pneumatikzylinder 15 angeordnet ist, durch welchen die Auflagekraft F_{A} des Reibkörpers 1 auf der Farb- oder Lackschicht 4 automatisch einstellbar ist.

Die in Figur 4d gezeigte Ausführungsform unterscheidet sich dadurch von der in Figur 4a gezeigten Ausführungsform, dass der Reibkörper 1 an Stelle des vertikal beweglichen Schafts fest mit einem im Drehpunkt 17 drehbar aufgehängten "Wägebalkens" 16 verbunden ist. Die Anpassung des Reibkörpers an das Substrat erfolgt hier nicht in vertikaler Richtung sondern auf einer Kreisbahn um den Drehpunkt 17. Die Auflagekraft F_{A} des Reibkörpers 1 auf der Farb- oder Lackschicht 4 ist entsprechend Figur 4a-c über Gewichte, Federn oder Pneumatikzylinder einstellbar.

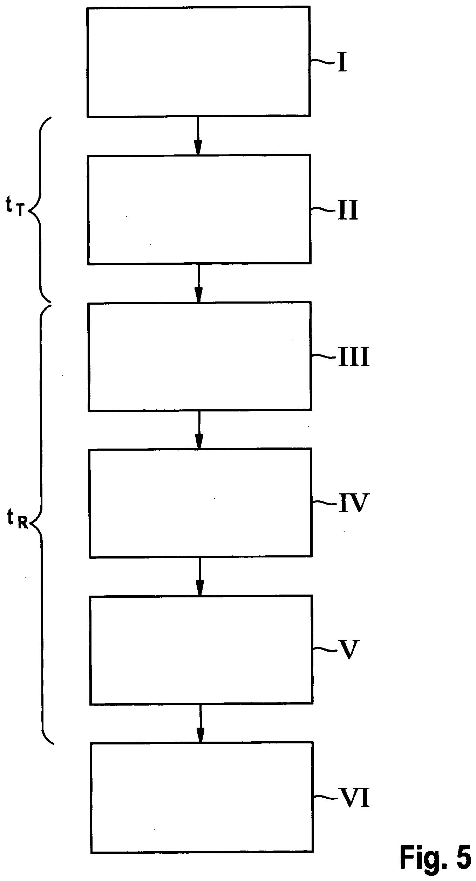

Figur 5 ist ein Flussdiagramm zur Veranschaulichung einer Ausführungsform des erfindungsgemäßen Verfahrens. Figur 5 veranschaulicht, dass in einem ersten Verfahrensschritt I eine Farb- oder Lackschicht automatisch, insbesondere durch ein Rakel-, Spritz-, Walz- und/oder Schleuder-Verfahren, auf ein Substrat, beispielsweise eine Prüfkarte, aufgebracht wird. Im zweiten Verfahrensschritt II wird die Bewegung eines Reibkörpers durch eine Reibkörperbewegungseinrichtung gestartet, wobei der Reibkörper zu diesem Zeitpunkt noch nicht in Reibkontakt mit einer Farb- oder Lackschicht steht. Nach einer definierten Wartezeit/Trocknungszeit t_{T} ab dem Aufbringen der Farb- oder Lackschicht im ersten Verfahrensschritt I wird der Reibkörper in einem dritten Verfahrensschritt III während der andauernden Bewegung automatisch, insbesondere mit einer definierten Auflagekraft (F_{A}), in Kontakt mit dem Prüfbereich der Farb- oder Lackschicht gebracht. Während eines vierten Verfahrensschrittes IV wird der Reibkörper in Reibkontakt entlang einer definierten Bahn über den Prüfbereich automatisch bewegt. In einem fünften Verfahrensschritt V wird der Reibkontakt zwischen dem Reibkörper und dem Prüfbereich während der andauernden Bewegung des Reibkörpers 1 automatisch, beispielsweise durch Herausbewegen des Reibkörpers aus dem Prüfbereich und/oder durch Abheben des Reibkörpers vom Prüfbereich, aufgehoben. Die beim Reibkontakt seitlich auf den Reibkörper wirkende Kraft (F_{R}) kann dabei gemessen und als Maß für den Trocknungszustand der Farb-oder Lackschicht und/oder als Maß für das Aufheben des Reibkontaktes verwendet werden. Figur 5 veranschaulicht, dass der Reibkörper vom Aufsetzen des Reibkörpers auf die Farb- oder Lackschicht in Verfahrensschritt III bis zum Aufheben des Reibkontaktes in Verfahrensschritt V eine definierte Reibzeit t_{R} lang in Reibkontakt über den Prüfbereich bewegt wird. Nach dem fünften Verfahrensschritt V kann der Reibkörper in einem sechsten Verfahrensschritt IV entweder automatisch gereinigt oder gegen einen neuen Einweg- Reibkörper ausgetauscht werden.

## Patentansprüche

1. Prüfvorrichtung zur Überprüfung der Qualität einer Farb- oder Lackschicht, umfassend mindestens eine Reibkörperbewegungseinrichtung, welche dazu ausgebildet ist, mindestens einen Reibkörper (1) in Reibkontakt entlang einer definierten Bahn (2) über einen Prüfbereich (3) einer Farb- oder Lackschicht (4) zu bewegen und den Reibkontakt zwischen dem Reibkörper (1) und dem Prüfbereich (3) während der Bewegung des Reibkörpers (1) aufzuheben,
**dadurch gekennzeichnet, dass** die Reibkörperbewegungseinrichtung derart ausgebildet ist, dass die Reibkörperbahn einen ersten, gekrümmten Bahnabschnitt im Prüfbereich und einen zweiten gradlinigen Bahnabschnitt aus dem Prüfbereich heraus umfasst, wobei der erste, gekrümmte Bahnabschnitt spiralförmig ist oder die Form eines einfach oder mehrfach durchlaufenen Ovals, insbesondere Kreises oder Ellipse, aufweist, wobei der zweite, gradlinige Bahnabschnitt ein Bahnabschnitt ist, welcher tangential von dem ersten, gekrümmten Bahnabschnitt ausgeht.

2. Prüfvorrichtung nach Anspruch 1, **dadurch gekennzeichnet, dass** die Reibkörperbewegungseinrichtung dazu ausgebildet ist, den Reibkörper (1) während der Bewegung
- aus dem Prüfbereich (3) heraus zu bewegen, und/oder
- von dem Prüfbereich (3) abzuheben.

3. Prüfvorrichtung nach Anspruch 1, **dadurch gekennzeichnet, dass** die Reibkörperbewegungseinrichtung dazu ausgebildet ist, den Reibkörper (1) in Reibkontakt entlang einer definierten, zumindest teilweise gekrümmten Bahn (2) über einen Prüfbereich (3) einer unbewegten Farb- oder Lackschicht (4) zu bewegen.

4. Prüfvorrichtung nach einem der Ansprüche 1 bis 3, **dadurch gekennzeichnet, dass** die Reibkörperbewegungseinrichtung derart ausgebildet ist, dass
- die Form der Reibkörperbahn (2) einstellbar ist, und/oder
- die Auflagekraft (F_{A}), mit welcher der Reibkörper (1) auf der Farb- oder Lackschicht (4) aufliegt, einstellbar ist, und/oder
- die Reibzeit (t_{R}), während welcher der Reibkörper (1) in Reibkontakt mit dem Prüfbereich (3) steht, einstellbar ist.

5. Prüfvorrichtung nach einem der Ansprüche 1 bis 4, **dadurch gekennzeichnet, dass** die Reibkörperbewegungseinrichtung mindestens einen Greifer (10), insbesondere einen Sauggreifer, zum lösbaren Befestigen eines Reibkörpers (1) umfasst.

6. Prüfvorrichtung nach einem der Ansprüche 1 bis 5, **dadurch gekennzeichnet, dass** der Reibkörper (1) ein Körper mit einer konvexen Reibfläche, insbesondere eine ausgewölbte Scheibe, ein Ovaloid oder ein Halbovaloid, ist.

7. Prüfvorrichtung nach einem der Ansprüche 1 bis 6, **dadurch gekennzeichnet, dass** der Reibkörper (1) reinigbar, insbesondere automatisch reinigbar, oder ein Einweg-Teil ist.

8. Prüfvorrichtung nach einem der Ansprüche 1 bis 7, **dadurch gekennzeichnet, dass** die Reibkörperbewegungseinrichtung dazu ausgebildet ist, den Reibkörper (1) während einer Bewegung des Reibkörpers (1) auf den Prüfbereich (3) der Farb- oder Lackschicht (4) aufzusetzen.

9. Prüfvorrichtung nach einem der Ansprüche 1 bis 8, **dadurch gekennzeichnet, dass** die Reibkörperbewegungseinrichtung mindestens einen Kraftsensor zum Messen einer beim Reibkontakt auf den Reibkörper seitlich wirkenden Kraft (F_{R}) umfasst.

10. Prüfvorrichtung nach einem der Ansprüche 1 bis 9, **dadurch gekennzeichnet, dass** die Reibkörperbewegungseinrichtung dazu ausgebildet ist, mindestens einen ersten und mindestens einen zweiten Reibkörper jeweils in Reibkontakt entlang einer definierten Bahn (2,2') über einen Prüfbereich (3,3') zu bewegen.

11. Prüfvorrichtung nach einem der Ansprüche 1 bis 10, **dadurch gekennzeichnet, dass** die Prüfvorrichtung zwei oder mehr Reibkörperbewegungseinrichtungen umfasst, wobei die Reibkörperbewegungseinrichtungen jeweils dazu ausgebildet sind, mindestens einen Reibkörper in Reibkontakt entlang einer definierten Bahn (2,2') über einen Prüfbereich (3,3') einer Farb- oder Lackschicht (4,4') zu bewegen und den Reibkontakt zwischen dem Reibkörper und dem Prüfbereich (3,3') während der Bewegung des Reibkörpers aufzuheben.

12. Prüfverfahren zur Überprüfung der Qualität einer Farb- oder Lackschicht mit einer Prüfvorrichtung nach einem der Ansprüche 1 bis 11, in dem
- mindestens ein Reibkörper (1) in Reibkontakt entlang einer definierten Bahn (2) über einen Prüfbereich (3) einer Farb- oder Lackschicht (4) bewegt wird und
- der Reibkontakt zwischen dem Reibkörper (1) und dem Prüfbereich (3) während der Bewegung des Reibkörpers (1) aufgehoben wird,
wobei die Reibkörperbahn einen ersten, gekrümmten Bahnabschnitt im Prüfbereich und einen zweiten gradlinigen Bahnabschnitt aus dem Prüfbereich heraus umfasst, wobei der erste, gekrümmte Bahnabschnitt spiralförmig ist oder die Form eines einfach oder mehrfach durchlaufenen Ovals, insbesondere Kreises oder Ellipse, aufweist, wobei der zweite, gradlinige Bahnabschnitt ein Bahnabschnitt ist, welcher tangential von dem ersten, gekrümmten Bahnabschnitt ausgeht.

13. Prüfverfahren nach Anspruch 12, **dadurch gekennzeichnet, dass** der Reibkontakt zwischen dem Reibkörper (1) und dem Prüfbereich (3) aufgehoben wird, in dem der Reibkörper (1) während der Bewegung,
- aus dem Prüfbereich (3) heraus bewegt, und/oder
- von dem Prüfbereich (3) abgehoben,

14. Prüfverfahren nach Anspruch 12 oder 13, **dadurch gekennzeichnet, dass**
- die Farb- oder Lackschicht (4) automatisch, insbesondere durch ein Rakel-, Spritz-, Walz- und/oder Schleuder-Verfahren, auf ein Substrat, insbesondere eine Prüfkarte, aufgebracht wird.
- der Reibkörper (1) nach einer definierten Wartezeit (t_{T}), ab dem Aufbringen der Farb- oder Lackschicht (4), automatisch, insbesondere mit einer definierten Auflagekraft (F_{A}), in Kontakt mit dem Prüfbereich (3) gebracht wird,
- der Reibkörper (1) eine definierte Reibzeit (t_{R}) lang in Reibkontakt über den Prüfbereich (3) bewegt wird.

15. Prüfverfahren nach einem der Ansprüche 12 bis 14, **dadurch gekennzeichnet, dass** die beim Reibkontakt seitlich auf den Reibkörper (11) wirkende Kraft (F_{R}) gemessen wird und als Maß für den Zustand, insbesondere den Trocknungszustand, der Farb- oder Lackschicht (4) und/oder als Maß für das Aufheben des Reibkontaktes verwendet wird.

## Claims

1. Test device for testing the quality of a paint or varnish layer, comprising at least one friction body movement device which is designed to move a friction body (1) in frictional contact along a defined path (2) over a test area (3) of a paint or varnish layer (4), and to cancel the frictional contact between the friction body (1) and the test area (3) during the movement of the friction body (1),
**characterized in that** the friction body movement device is designed in such a way that the friction body path comprises a first, curved path section in the test area and a second, straight path section out of the test area, wherein the first, curved path section is spiral-shaped or has the shape of an oval, in particular a circle or ellipse, passed through once or repeatedly, wherein the second, straight path section is a path section which extends tangentially from the first, curved path section.

2. Test device according to Claim 1, **characterized in that** the friction body movement device is designed
- to move the friction body (1) out of the test area (3), and/or
- to lift the friction body (1) off the test area (3)
during the movement.

3. Test device according to Claim 1, **characterized in that** the friction body movement device is designed to move the friction body (1) in frictional contact along a defined, at least partly curved, path (2) over a test area (3) of an unmoved paint or varnish layer (4).

4. Test device according to one of Claims 1 to 3, **characterized in that** the friction body movement device is designed such that
- the shape of the friction body path (2) is adjustable, and/or
- the contact force (F_{A}) with which the friction body (4) rests on the paint or varnish layer (4) is adjustable, and/or
- the friction time (t_{R}) during which the friction body (1) is in frictional contact with the test area (3) is adjustable.

5. Test device according to one of Claims 1 to 4, **characterized in that** the friction body movement device comprises at least one gripper (10), in particular a suction gripper, for the detachable fixing of a friction body (1).

6. Test device according to one of Claims 1 to 5, **characterized in that** the friction body (1) is a body having a convex frictional surface, in particular a convexly curved disc, an ovoid or a semi-ovoid.

7. Test device according to one of Claims 1 to 6, **characterized in that** the friction body (1) can be cleaned, in particular cleaned automatically, or is a disposable part.

8. Test device according to one of Claims 1 to 7, **characterized in that** the friction body movement device is designed to set the friction body (1) onto the test area (3) of the paint or varnish layer (4) during a movement of the friction body (1).

9. Test device according to one of Claims 1 to 8, **characterized in that** the friction body movement device comprises at least one force sensor for measuring a force (F_{R}) acting laterally on the friction body during the frictional contact.

10. Test device according to one of Claims 1 to 9, **characterized in that** the friction body movement device is designed to move at least one first and at least one second friction body respectively along a defined path (2, 2') over a test area (3, 3').

11. Test device according to one of Claims 1 to 10, **characterized in that** the test device comprises two or more friction body movement devices, wherein the friction body movement devices are each designed to move at least one friction body in frictional contact along a defined path (2, 2') over a test area (3, 3') of a paint or varnish layer (4, 4'), and to cancel the frictional contact between the friction body and test area (3, 3') during the movement of the friction body.

12. Test method for testing the quality of a paint or varnish layer using a test device according to one of Claims 1 to 11, in which
- at least one friction body (1) is moved in frictional contact along a defined path (2) over a test area (3) of a paint or varnish layer (4), and
- the frictional contact between the friction body (1) and the test area (3) is cancelled during the movement of the friction body (1),
wherein the friction body path comprises a first, curved path section in the test area and a second, straight path section out of the test area, wherein the first, curved path section is spiral-shaped or has the shape of an oval, in particular a circle or ellipse, passed through once or repeatedly, wherein the second, straight path section is a path section which extends tangentially from the first, curved path section.

13. Test method according to Claim 12, **characterized in that** the frictional contact between the friction body (1) and the test area (3) is cancelled **in that**, during the movement, the friction body (1)
- moved out of the test area (3), and/or
- lifted off the test area (3),

14. Test method according to Claim 12 or 13, **characterized in that**
- the paint or varnish layer (4) is applied to a substrate, in particular a test card, automatically, in particular by means of a doctoring, spraying, rolling and/or centrifugal method,
- the friction body (1) is brought into contact with the test area (3) automatically, in particular with a defined contact force (F_{A}), after a defined waiting time (t_{T}) beginning from the application of the paint or varnish layer (8),
- the friction body (1) is moved in frictional contact over the test area (3) for a defined friction time (t_{R}).

15. Test method according to one of Claims 12 to 14, **characterized in that** the force (F_{R}) acting laterally on the friction body (11) during the frictional contact is measured and is used as a measure of the state, in particular the state of drying, of the paint or varnish layer (4) and/or as a measure for the cancellation of the frictional contact.

## Revendications

1. Dispositif de contrôle pour vérifier la qualité d'une couche de peinture ou de laque, comprenant au moins un dispositif de déplacement de corps de friction qui est réalisé pour déplacer au moins un corps de friction (1) en contact de friction le long d'une piste définie (2) sur une région de contrôle (3) d'une couche de peinture ou de laque (4) et pour supprimer le contact de friction entre le corps de friction (1) et la région de contrôle (3) pendant le déplacement du corps de friction (1), **caractérisé en ce que** le dispositif de déplacement de corps de friction est configuré de telle sorte que la piste de corps de friction possède une première portion de piste courbe dans la région de contrôle et une deuxième portion de piste rectiligne hors de la région de contrôle, la première portion de piste courbe étant en forme de spirale ou présentant la forme d'un ovale parcouru une ou plusieurs fois, notamment un cercle ou une ellipse, la deuxième portion de piste rectiligne étant une portion de piste qui affleure la première portion de piste courbe de manière tangentielle.

2. Dispositif de contrôle selon la revendication 1, **caractérisé en ce que** le dispositif de déplacement de corps de friction est réalisé de manière à, pendant le déplacement,
- déplacer le corps de friction (1) hors de la région de contrôle (3), et/ou
- à le soulever de la région de contrôle (3).

3. Dispositif de contrôle selon la revendication 1, **caractérisé en ce que** le dispositif de déplacement de corps de friction est réalisé de manière à déplacer le corps de friction (1) en contact de friction le long d'une piste définie. (2) au moins partiellement courbe sur une région de contrôle (3) d'une couche de peinture ou de laque (4) non déplacée.

4. Dispositif de contrôle selon l'une quelconque des revendications 1 à 3, **caractérisé en ce que** le dispositif de déplacement de corps de friction est réalisé de telle sorte que
- la forme de la piste de corps de friction (2) puisse être ajustée, et/ou
- la force d'appui (F_{A}) avec laquelle le corps de friction (1) repose sur la couche de peinture ou de laque (4) puisse être ajustée, et/ou
- la durée de friction (t_{R}) pendant laquelle le corps de friction (1) est en contact de friction avec la région de contrôle (3) puisse être ajustée.

5. Dispositif de contrôle selon l'une quelconque des revendications 1 à 4, **caractérisé en ce que** le dispositif de déplacement de corps de friction comprend au moins un dispositif de préhension (10), en particulier un dispositif de préhension à aspiration, pour la fixation amovible d'un corps de friction (1).

6. Dispositif de contrôle selon l'une quelconque des revendications 1 à 5, **caractérisé en ce que** le corps de friction (5) est un corps avec une surface de friction convexe, en particulier un disque cintré, un ovaloïde ou un semi-ovaloïde.

7. Dispositif de contrôle selon l'une quelconque des revendications 1 à 6, **caractérisé en ce que** le corps de friction (1) peut être nettoyé, en particulier peut être nettoyé automatiquement, ou est une pièce à usage unique.

8. Dispositif de contrôle selon l'une quelconque des revendications 1 à 7, **caractérisé en ce que** le dispositif de déplacement de corps de friction est réalisé de manière à poser le corps de friction (1) pendant un déplacement du corps de friction (1) sur la région de contrôle (3) de la couche de peinture ou de laque (4).

9. Dispositif de contrôle selon l'une quelconque des revendications 1 à 8, **caractérisé en ce que** le dispositif de déplacement de corps de friction comprend au moins un capteur de force pour mesurer une force (F_{R}) agissant latéralement sur le corps de friction lors du contact de friction.

10. Dispositif de contrôle selon l'une quelconque des revendications 1 à 9, **caractérisé en ce que** le dispositif de déplacement de corps de friction est réalisé de manière à déplacer au moins un premier et au moins un deuxième corps de friction à chaque fois en contact de friction le long d'une piste définie (2, 2') sur une région de contrôle (3, 3').

11. Dispositif de contrôle selon l'une quelconque des revendications 1 à 10, **caractérisé en ce que** le dispositif de contrôle comprend deux ou plus de deux dispositifs de déplacement de corps de friction, les dispositifs de déplacement de corps de friction étant réalisés à chaque fois de manière à déplacer au moins un corps de friction en contact de friction le long d'une piste définie (2, 2') sur une région de contrôle (3, 3') d'une couche de peinture ou de laque (4, 4') et à supprimer le contact de friction entre le corps de friction et la région de contrôle (3, 3') pendant le déplacement du corps de friction.

12. Procédé de contrôle pour vérifier la qualité d'une couche de peinture ou de laque avec un dispositif de contrôle selon l'une des revendications 1 à 11, dans lequel
- au moins un corps de friction (1) est déplacé en contact de friction le long d'une piste définie (2) sur une région de contrôle (3) d'une couche de peinture ou de laque (4) et
- le contact de friction entre le corps de friction (1) et la région de contrôle (3) est supprimé pendant le déplacement du corps de friction (1), la piste de corps de friction possédant une première portion de piste courbe dans la région de contrôle et une deuxième portion de piste rectiligne hors de la région de contrôle, la première portion de piste courbe étant en forme de spirale ou présentant la forme d'un ovale parcouru une ou plusieurs fois, notamment un cercle ou une ellipse, la deuxième portion de piste rectiligne étant une portion de piste qui affleure la première portion de piste courbe de manière tangentielle.

13. Procédé de contrôle selon la revendication 12, **caractérisé en ce que** le contact de friction entre le corps de friction (1) et la région de contrôle (3) est supprimé **en ce que** le corps de friction (1), pendant le déplacement,
- déplacé hors de la région de contrôle (3), et/ou
- soulevé de la région de contrôle (3),

14. Procédé de contrôle selon la revendication 12 ou 13, **caractérisé en ce que**
- la couche de peinture ou de laque (4) est appliquée automatiquement, en particulier par un procédé de raclage, de pulvérisation, d'enduction au rouleau et/ou de centrifugation, sur un substrat, en particulier une carte de contrôle,
- le corps de friction (1), après un temps d'attente défini (t_{T}), à partir de l'application de la couche de peinture ou de laque (4), est amené automatiquement, en particulier avec une force d'appui définie (F_{A}), en contact avec la région de contrôle (3),
- le corps de friction (1) est déplacé pendant une durée de friction définie (t_{R}) en contact de friction sur la région de contrôle (3).

15. Procédé de contrôle selon l'une quelconque des revendications 12 à 14, **caractérisé en ce que** la force (F_{R}) agissant lors du contact de friction latéralement sur le corps de friction (11) est mesurée et est utilisée en tant que mesure d'état, en particulier de l'état de séchage, de la couche de peinture ou de laque (4) et/ou en tant que mesure de la suppression du contact de friction.
